(19) Europäisches Patentamt
European Patent Office
Office européen des brevets

(11) Numéro de publication: **0 434 571 B1**

(12) **FASCICULE DE BREVET EUROPEEN**

(45) Date de publication de fascicule du brevet: **01.02.95**

(51) Int. Cl.$^{6}$: **C07J 41/00**, C07J 1/00, A61K 31/56

(21) Numéro de dépôt: **90403727.2**

(22) Date de dépôt: **21.12.90**

Le dossier contient des informations techniques présentées postérieurement au dépôt de la demande et ne figurant pas dans le présent fascicule.

# (54) Nouveaux produits stéroides comportant, en position 10, un radical éthyle substitué, leur procédé de préparation, application comme médicament de l'un de ces produits et les compositions pharmaceutiques le renfermant.

(30) Priorité: **22.12.89 FR 8917048**

(43) Date de publication de la demande: **26.06.91 Bulletin 91/26**

(45) Mention de la délivrance du brevet: **01.02.95 Bulletin 95/05**

(84) Etats contractants désignés: **BE CH DE FR GB IT LI NL**

(56) Documents cités:
**EP-A- 0 104 489**
**US-A- 4 096 254**

**Journal of Chemical Society (1962) page 1223;**

**Journal of the American Chemical Society (1960) page 4293;**

**Journal of the American Chemical Society (1980) pages 3270-2;**

**Helvetica Chimica Acta (1967), vol. 50(7) pages 2091-5**

(73) Titulaire: **ROUSSEL-UCLAF**
**35, Boulevard des Invalides**
**F-75007 Paris (FR)**

(72) Inventeur: **Gourvest, Jean-François**
**12, rue de la Biberonne**
**F-77410 Clave-Souilly (FR)**
Inventeur: **Lesuisse, Dominique**
**2, rue Cazotte**
**F-75018 Paris (FR)**

(74) Mandataire: **Bourgouin, André et al**
**Département des Brevets**
**ROUSSEL UCLAF**
**111, route de Noisy**
**B.P. no 9**
**F-93230 Romainville (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 434 571 B1

## Description

La présente invention concerne de nouveaux produits stéroïdes comportant, en position 10, un radical éthyle substitué, leur procédé de préparation, l'application comme médicament de l'un de ces produits et les compositions pharmaceutiques le renfermant.

L'invention a pour objet les produits de formule générale (I) :

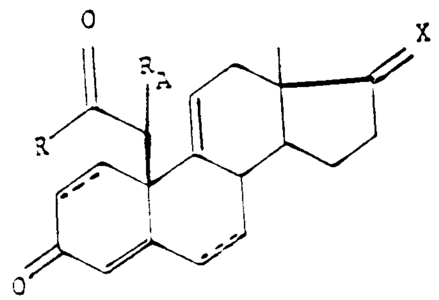

(I)

dans laquelle le substituant R représente :

- un atome d'hydrogène,
- un radical :

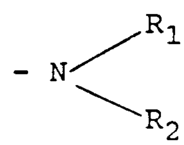

dans lequel $R_1$ et $R_2$ identiques ou différents représentent soit un radical alkyle ayant de 1 à 6 atomes de carbone soit $R_1$ et $R_2$ forment avec l'atome d'azote auquel ils sont liés un radical hétérocyclique à 5 ou 6 chaînons comportant éventuellement un second hétéroatome choisi parmi les atomes d'oxygène, de soufre et d'azote,

- un radical alkyle ou alkyloxy ayant chacun de 1 à 12 atomes de carbone,
- un radical aryle, aralkyle, aryloxy ou (arylalkyl) oxy ayant chacun au plus 12 atomes de carbone,

$R_A$ représente un atome d'hydrogène, un atome d'halogène, un radical hydroxy, un radical alkyle, alkyloxy, alkylthio, amino, monoalkylamino, dialkylamino, dans chacun desquels alkyl renferment au plus 6 atomes de carbone, un radical carbamoyle, un radical alkoxycarbonyle renfermant au plus 7 atomes de carbone,

X représente un atome d'oxygène ou un radical

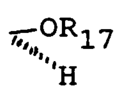

dans lequel $R_{17}$ représente un atome d'hydrogène ou un radical alkyle ou acyle ayant chacun au plus 12 atomes de carbone, et les traits pointillés en positions 1(2) et 6(7) indiquent la présence éventuelle d'une seconde liaison entre les carbones qui les portent, étant entendu que R représente un radical alkyloxy, aryloxy, ou (arylalkyl)oxy ayant chacun au plus 12 atomes de carbone lorsque $R_A$ ne représente pas un atome d'hydrogène.

Parmi les radicaux alkyle ayant de 1 à 6 atomes de carbone, on peut citer les radicaux méthyle, éthyle, propyle, isopropyle, butyle, isobutyle, butyle secondaire, tertbutyle, pentyle, hexyle.

Parmi les radicaux alkyle ayant de 1 à 12 atomes de carbone, on peut citer en plus les radicaux heptyle, octyle, nonyle, décyle, indécyle, dodécyle linéaires ou ramifiés. Le radical :

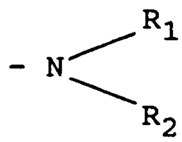

peut représenter les radicaux dialkyle amino, dans lesquels les radicaux alkyle ont les valeurs précédentes. On préfère les radicaux comportant deux radicaux alkyle identiques, de préférence le radical diméthylamino.

Le radical :

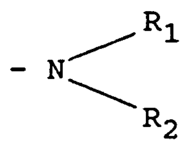

peut également représenter un radical hétérocyclique à 5 chaînons tel que le radical pyrrolidinyle ou un radical hétérocyclique à 6 chaînons tel que les radicaux pipéridinyle, morpholinyle, pipérazinyle éventuellement substitué sur l'atome d'azote par un radical alkyle tel que les radicaux méthyle et éthyle (on obtient alors les radicaux méthylpipérazinyle et éthylpipérazinyle).

Parmi les radicaux alkyloxy on peut citer les radicaux dérivés des radicaux alkyle cités ci-dessus. On préfère les radicaux méthoxy et surtout éthoxy.

Parmi les radicaux aryle on peut citer les radicaux aryle carbocycliques tels que le phényle ou le naphtyle ou les aryles hétérocycliques à 5 ou 6 chaînons comportant un ou plusieurs hétéroatomes choisis de préférence parmi l'oxygène, le soufre et l'azote. Parmi les aryles hétérocycliques à 5 chaînons, on peut citer les radicaux furyle, thiényle, pyrrolyle, thiazolyle, oxazolyle, imidazolyle, thiadiazolyle, pyrazolyle, isoxazolyle.

Parmi les aryles hétérocycliques à 6 chaînons, on peut citer les radicaux pyridyle, pyrimidinyle, pyridazinyle, pyrazinyle. Parmi les cycles condensés on peut citer les radicaux indolyle, benzofurannyle, benzothiényle, quinoléinyle. On préfère le radical phényle.

Les radicaux aryloxy sont dérivé des radicaux aryles mentionnés ci-dessus. On préfère le radical phénoxy.

Parmi les valeurs aralkyle on préfère les valeurs benzyle et phényléthyle. Parmi les valeurs aralkyloxy on préfère les valeurs benzyloxy et phényléthoxy. Parmi les valeurs de R on préfère les valeurs hydrogène, méthyle, éthoxy et diméthylamino, la valeur alkyle que peut représenter $R_{17}$ peut être choisie parmi les valeurs indiquées ci-dessus. On préfère les radicaux alkyle ayant de 1 à 6 atomes de carbone et notamment le radical méthyle.

Parmi les valeurs acyle que peut représenter $R_{17}$ on peut citer les radicaux acyle dérivés des acides carboxyliques. On préfère les radicaux acétyle, propionyle ou benzoyle.

Parmi les atomes d'halogène, on peut citer les atomes de chlore, de fluor ou de brome.

Parmi les radicaux alkylthio, on peut citer les radicaux correspondant aux radicaux alkyle mentionnés ci-dessus.

On peut citer par exemple le radical méthylthio ou éthylthio.

Parmi les radicaux alkoxycarbonyle, on peut citer les radicaux dérivés des radicaux alkyle cités ci-dessus.

On préfère les radicaux méthoxycarbonyle et surtout éthoxycarbonyle.

L'invention a plus particulièrement pour objet les produits de formule générale (I') :

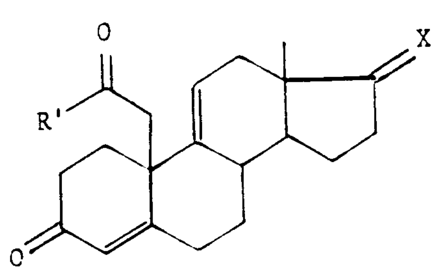

(I')

dans laquelle R' représente un atome d'hydrogène, un radical $-N(R'_1)_2$ dans lequel $R'_1$ représente un radical alkyle ayant de 1 à 4 atomes de carbone ou R' représente un radical alkyle ou alkyloxy ayant chacun de 1 à 4 atomes de carbone, X représente un atome d'oxygène ou un radical

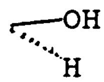

Parmi les produits de formule (I'), l'invention a particulièrement pour objet les produits décrits ci-après dans les exemples et en particulier le 3-17-dioxo androsta-4,9(11)-diène-19-carboxaldéhyde.

La présente invention a également pour objet un procédé de préparation des produits de formule générale (I) telle que définie ci-dessus et caractérisée en ce que l'on soumet un produit de formule (II) :

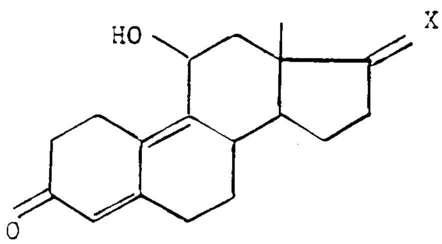

(II)

dans laquelle X a la signification indiquée ci-dessus à l'action :
soit d'un produit de formule (III) :

$R_A\text{-}CH_2\text{-}C(OR_a)_3$ (III)

dans laquelle $R_A$ a la signification indiquée ci-dessus $R_a$ représente un radical alkyle, aryle ou arylalkyle ayant chacun au plus 12 atomes de carbone, pour obtenir les produits de formule (Ia) correspondant aux produits de formule (I) dans laquelle R représente un radical alkyloxy, aryloxy ou (arylalkyl) oxy ayant chacun au plus 12 atomes de carbone, $R_A$ a la signification indiquée ci-dessus et les traits pointillés en positions 1(2) et 6(7) ne représentent pas une seconde liaison-entre les carbones qui les portent,
soit d'un produit de formule (IV) :

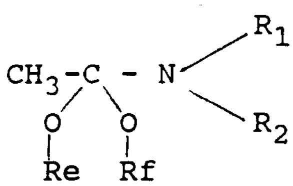

(IV)

dans laquelle $R_1$ et $R_2$ ont la signification indiquée ci-dessus Re et Rf identiques ou différents représentent un radical alkyle ayant de 1 à 4 atomes de carbone, pour obtenir les produits de formule (Ib) correspondant aux produits de formule (I) dans laquelle R représente :

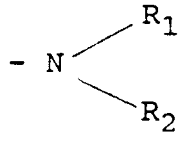

dans lequel $R_1$ et $R_2$ ont la signification indiquée ci-dessus, $R_A$ représente un atome d'hydrogène et les traits pointillés en position 1(2) et 6(7) ne représentent pas une seconde liaison entre les carbones qui les portent,
soit d'un produit de formule (V) :

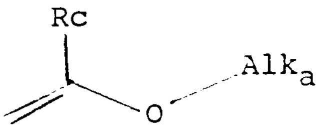

(V)

dans laquelle Rc représente un atome d'hydrogène ou un radical alkyle, aryle ou aralkyle ayant chacun au plus 12 atomes de carbone et $Alk_a$ représente un radical alkyle ayant de 1 à 4 atomes de carbone, pour obtenir les produits de formule (Ic) correspondant aux produits de formule (I) dans laquelle R représente un atome d'hydrogène ou un radical alkyle, aryle ou aralkyle ayant chacun au plus 12 atomes de carbone, $R_A$ représente un atome d'hydrogène et les traits pointillés en positions 1(2) et 6(7) ne représentent pas une seconde liaison entre les carbones qui les portent, produits de formules (Ia), (Ib) ou (Ic) que si désiré l'on soumet à l'une ou plusieurs des réactions suivantes, et dans un ordre quelconque :

- introduction d'une double liaison en position 1(2)
- introduction d'une double liaison en position 6(7)
- réduction de la fonction cétone en position 17 lorsque X représente un atome d'oxygène et formation d'un groupement 17$\beta$-OH
- éthérification ou estérification des produits dans lesquels X représente un radical

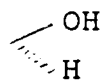

de façon à obtenir 17$\beta$-$OR_{17}$ dans lequel $R_{17}$ est un alkyle ou un acyle ayant chacun au plus 12 atomes de carbone.

Dans un mode préféré d'exécution du procédé ci-dessus,

- l'action du produit de formule (III) sur les produits de formule (II) est réalisée en présence d'un acide, par exemple un acide organique tel que les acides acétique, propionique, ou p-toluènesulfonique. On peut opérer au sein d'un solvant à haut point d'ébullition tel que le toluène ou le xylène,
- l'action du produit de formule (IV) est également effectuée au sein d'un solvant à haut point d'ébullition tel que le toluène ou le xylène cités ci-dessus. Dans la formule (IV), Re et Rf représentent de préférence chacun un radical méthyle ou éthyle ou forment ensemble un radical éthylène,
- l'action du produit de formule (V) est effectuée de préférence en présence d'acétate de mercure et à température élevée.

L'action des produits de formules (III), (IV) et (V) sur un produit de formule (II) donne naissance à ce qu'il est convenu d'appeler un réarrangement de Claisen,

- l'introduction éventuelle d'une double liaison en position 1(2) est effectuée par la DDQ de préférence dans un solvant tel que le dioxanne (J. Chem. Soc. (1962) p 1223),
- l'introduction éventuelle d'une double liaison en position 6(7) est effectuée à l'aide d'un orthoformiate tel que l'orthoformiate d'éthyle en présence par exemple d'acide paratoluène sulfonique ou camphorsulfonique dans un solvant usuel tel que l'éthanol réaction suivie par celle de chloranile dans un solvant tel que l'acétone aqueux ou le tétrahydrofuranne aqueux (JACS, 82, p4293 (1960)),
- la réduction éventuelle de la fonction cétone en position 17 est effectuée à l'aide d'un hydrure tel que le borohydrure de sodium dans un solvant tel que le méthanol,
- l'éthérification éventuelle de l'alcool en position 17béta est effectuée par action d'abord d'une base forte telle que le tert-butylate de potassium ou l'hydrure de potassium suivie de l'action d'un halogénure ou d'un pseudo-halogénure dérivé du radical $R_{17}$ de formule $R_{17}$-X dans laquelle X représente de préférence un atome de chlore ou de brome ou un radical mésyle ou tosyle, la réaction est effectuée de préférence dans un solvant polaire. L'estérification éventuelle de l'alcool en position 17béta est effectuée de préférence à l'aide d'un dérivé réactif du radical acyle tel qu'un halogénure d'acide, de préférence le chlorure d'acide, un anhydride mixte ou symétrique. On peut également opérer avec l'acide carboxylique correspondant au radical acyle que l'on veut introduire en utilisant un agent de déshydratation tel que le dicyclohexyle carbodiimide (DCC).

L'observation selon laquelle environ 35 % des cancers du sein sont estrogéno dépendants a conduit à rechercher les moyens de limiter la production d'estrogènes.

Après avoir utilisé des méthodes chirurgicales consistant à supprimer les sources d'estrogènes (ovaires) ou les sources de leurs précurseurs biosynthétiques, les androgènes (glandes surrénales) on a cherché à développer des méthodes moins traumatisantes.

(ABUL-HAJJ Y.J.O. Steroid Biochem 13 (1980), 1935 ; BRODIE A.M.H. Cancer Res. 42, (1982), 3312).

A cet égard l'inhibition spécifique du dernier stade enzymatique de l'aromatisation des androgènes 3-céto delta-4 en estrogènes phénoliques, semble le moyen le plus efficace et le moins perturbant. L'enzyme responsable de cette transformation est une mono-oxygénase connue comme étant un cytochrome P450 : l'AROMATASE (BRODIE A.M.H. J. Endocrinol. Invest. 2 (1979) 445) qui requiert de l'oxygène et la NADPH (Nicotinamide Adenine Dinucleotide phosphate réduit) pour effectuer l'aromatisation des androgènes en estrogènes.

Sur la base d'un autre mécanisme, d'autres auteurs (par exemple MARCOTTE et Al, Biochemistry 21, (1982) 2773, FLYNN et Al Biochem. Biophys. Res. Com. 103 (1981) 713) ont proposé des inhibiteurs suicides pour l'Aromatase. Des inhibiteurs compétitifs tels que l'Aminogluthétimide ont également été proposés dans le traitement des cancers métastasiques du sein. Ce produit s'est cependant révélé comme n'étant pas spécifique de l'Aromatase : il s'attaque en effet à d'autres processus enzymatiques que celui conduisant des androgènes aux estrogènes.

Les produits objets de la présente invention présentent au contraire une activité spécifique de l'aromatase (cytochrome P450 aromatase).

Cette propriété inhibitrice de l'aromatase rend les produits de la présente invention aptes à être utilisés dans les affections suivantes :

- cancers du sein, de l'endomètre, de l'ovaire et du pancréas,
- gynécomasties,
- troubles bénins du sein,
- endométriose,
- affections polycystiques de l'ovaire,
- hyperplasie prostatique et plus généralement dans le traitement des hyperestrogènémies.

L'invention a tout spécialement pour objet, à titre de médicament le 3-17-dioxo androsta-4,9(11)-diène-19-carboxaldéhyde.

La posologie varie en fonction de l'affection à traiter et de la voie d'administration. Elle peut varier par exemple de 0,1 à 50 mg/kg de préférence 0,5 à 10 mg/kg et par jour chez l'adulte par voie orale.

Le produit de formule (I) 3-17-dioxo androsta-4,9(11)-diène-19-carboxaldéhyde peut être employé pour préparer des compositions pharmaceutiques renfermant, à titre de principe actif, ce produit.

Le produit de formule (I) 3-17-dioxo androsta-4,9(11)-diène-19-carboxaldéhyde est utilisé par voie digestive, parentérale ou locale par exemple par voie transdermique. Il peut être prescrit sous forme de comprimés simples ou dragéifiés, de gélules, de granulés, de suppositoires, d'ovules, de préparations injectables, de pommades, de crèmes, de gels, de patches, lesquels sont préparés selon les méthodes usuelles.

Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a donc pour objet les compositions pharmaceutiques renfermant comme principe actif le 3-17-dioxo androsta-4,9(11)-diène-19-carboxaldéhyde.

Les produits de formule (II) sont décrits, par exemple dans les brevets américains USP 3.211.764 et 3.282.785.

Les produits de formule (III) et (V) sont ou bien connus de l'homme du métier ou peuvent être préparés par des méthodes connues de l'homme de métier. On peut notamment citer les références suivantes :

- J. Gen. Chem. 6, 576-83 (1936),
- Chem. Abst. 30, 5583 (1936),
- Chem. Abst. 41, p. 5904a (1947),
- Wh. Graham. Tet. Lett. 27, 2223 (1969),
- JACS. 64, 1825.7 (1942),
- JACS. 102, p3270-2 (1980),
- Helv. Chim. Acta 50(7), p2091-5 (1967)

D'une manière générale, les produits de formule (III) peuvent être préparés à partir de produits de type $R_A$-$CH_2$-C≡N.

Les exemples suivants illustrent l'invention sans toutefois la limiter.

**EXEMPLE 1 : 3,17-dioxo androsta-4,9(11)-diène-19-carboxylate d'éthyle.**

On amène à une température de 137°C, un mélange de 500 mg de 11béta-hydroxy androsta-4,9-diène-3,17-dione (produit décrit dans le brevet américain USP 3.282.785) 5 $cm^3$ d'orthoacétate de triéthyle et 6,4 mg d'acide propionique.

Après 4 heures de chauffage, la réaction est concentrée jusqu'à sec et le mélange brut est ensuite chromatographié sur silice avec un mélange éluant d'acétate-éthyle hexane 1/1). On obtient 503 mg de produit attendu (Rf = 0,33).

RMN ($CDCl_3$, 250 MHz) 0,94 (s, 18-Me), 1,23 (t, $COOCH_2\underline{CH_3}$), 3,94 à 4,29 (m, $COO\underline{CH_2}$ $CH_3$), 5,61 (m, H11), 5,84 (large s, H14).

IR ($CHCl_3$) 1732 (17-cétone), 1662, 1612 (cétone conjuguée).

**EXEMPLE 2 : N,N-diméthyl 3,17-dioxo androsta-4,9(11)-diène-19-carboxamide.**

On porte au reflux pendant 2 heures en mélange de 500 mg de 11béta-hydroxy androsta-4,9-diène-3,17-dione, 350 mg de diméthylacétamide diméthylacétal et 30 $cm^3$ de toluène.

On évapore le solvant sous pression réduite, le produit brut cristallise dans l'éther isopropylique. On obtient ainsi 495 mg de produit attendu que l'on purifie par recristallisation dans l'acétate d'éthyle.

RMN ($CDCl_3$, 300 MHz) 0,89 (s, 18-Me), 2,72 (m, 19-$CH_2$), 2,90 et 3,06 (CONMe2), 5,71 (H11), 5,87 (H4).

IR (CHCl3) 1735 $cm^{-1}$ (17-cétone), 1665 $cm^{-1}$ (cétone conjuguée), 1643 $cm^{-1}$ (amide tertiaire).

| Microanalyse $C_{22}H_{29}O_3N$ = 355,46 | | | | | | |
|---|---|---|---|---|---|---|
| Calculé<br>Trouvé | C% | 74,33<br>74,1 | H% | 8,22<br>8,1 | N% | 3,94<br>3,7 |

**EXEMPLE 3 : 3,17-dioxo androsta-4,9(11)-diène-19-carboxaldéhyde.**

On chauffe à 80°C pendant 12 heures, un mélange de 4,5 g de 11béta-hydroxy androsta-4,9-diène-3,17-dione, 1,25 g d'acétate de mercure et 18 $cm^3$ d'éthyle vinyle éther. On verse dans un mélange 1/1 de dichlorométhane et de chlorure d'ammonium. La phase aqueuse est extraite au dichlorométhane. Ces extraits organiques sont séchés sur sulfate de magnésium et concentrés. On obtient 6,06 g de produit brut. Une précipitation dans l'éther fournit 3,79 g d'aldéhyde recherché que l'on recristallise dans l'éthanol et séche à 150°C sous pression réduite en présence de pentoxyde de phosphore.

RMN ($CDCl_3$, 250MHz) 0,85 (s, 18-Me), 5,70 (H11), 5,87 (H4), 9,66 (t, CHO).

IR ($CHCl_3$) 1736 et 1406 $cm^{-1}$ (17-cétone), 1721 et 2740 $cm^{-1}$ (CHO), 1671 et 1615 $cm^{-1}$ (cétone conjuguée), 1632 $cm^{-1}$ (C=C).

| Microanalyse $C_{20}H_{24}O_3$ = 312,39 | | | | |
|---|---|---|---|---|
| Calculé<br>Trouvé | C% | 76,89<br>76,7 | H% | 7,74<br>8,0 |

**EXEMPLE 4 : 10béta-(2-oxopropyl) estra-4,9(11)-diène-3,17-dione.**

On chauffe à 80°C pendant 12 heures, un mélange de 3 g de 11béta-hydroxy androsta-4,9-diène-3,17-dione, 834 mg d'acétate de mercure et 12 $cm^3$ de méthyl isopropényle éther. On verse dans un mélange de dichlorométhane et de chlorure d'ammonium.

La phase aqueuse est extraite au dichlorométhane. Les extraits organiques sont rassemblés, séchés et concentrés. Le mélange brut est chromatographié sur silice (éluant: acétate d'éthyle/hexane 1:1). On obtient 390 mg de produit attendu (Rf = 0,21) que l'on recristallise dans un mélange éther/éthanol.

RMN ($CDCl_3$, 250 MHz) 0,91 (s, 18-Me), 2,16 (s, $COCl_3$) 2,76 et 2,88 (2d, J=15, $CH_2CO$), 5,66 (m, H11) 5,83 (s, H4).

IR (CHCl3) 1717 et 1705 $cm^{-1}$ ($COCH_3$), 1735 $cm^{-1}$ (17-céto) 1669 et 1615 $cm^{-1}$ (3-céto).

| Microanalyse $C_{21}H_{26}O_3$ = 326,42 | | | | |
|---|---|---|---|---|
| Calculé<br>Trouvé | C% | 77,27<br>71,10 | H% | 8,03<br>8,0 |

**EXEMPLE 5 : N,N-diméthyl 17béta-hydroxy androsta-4,9(11)-dièn-3-one-19-carboxamide.**

On porte au reflux pendant 3 heures un mélange de 1 g de 11béta, 17béta-diol androsta-4,9-dièn-3-one, 1,158 g de diméthylacétal de diméthylacétamide et 50 $cm^3$ de toluène.

On concentre sous pression réduite et chromatographie sur silice (éluant: acétate d'éthyle-méthanol 9-1). On obtient ainsi 930 mg de produit attendu.

IR ($CHCl_3$) : 1642 $cm^{-1}$ (amide tertiaire) 1662, 880 et 869 $cm^{-1}$ (cétone conjuguée).

SM : 357 ($M^+$).

**EXEMPLE 6 : 3,17-dioxo androsta 4,9(11)-dièn 19-dicarboxylate d'éthyle.**

On chauffe 3 heures à 140 °C 300 mg de 11béta-hydroxy androsta 4,9-dièn 3,17-dione, 2 $cm^3$ d'orthomalonate de triéthyle et 10 μl d'acide propionique. On ajoute quelques gouttes de triéthylamine, concentre à sec sous pression réduite et chromatographie le résidu sur silice (éluant : acétate d'éthyle-cyclohexane 0-1 puis 3-7). On obtient 360 mg de produit attendu. Rf = 0,54 (cyclohexane-acétate d'éthyle 1-1).

IR ($CHCl_3$) : 1750 $cm^{-1}$ (esters); 1734 $cm^{-1}$ (17-céto) ; 1669 $cm^{-1}$ (cétone conjuguée) ; 1636 et 1620 $cm^{-1}$ (C=C).

RMN ($CDCl_3$, 300 MHz) : 0,87 (s, 18 Me) ; 1,22 (m, COOEt) ; 4,15 (m, COOEt) ; 4,26 (s, $CH(COOEt)_2$ ; 5,74 (m, H11) ; 5,91 (s, H4).

**EXEMPLE 7 : 3,17-dioxo androsta 4,9(11)-dièn 19-chloro 19-carboxylate d'éthyle.**

On chauffe 2 heures à 140 °C 300 mg de 11béta-hydroxy androsta 4,9-dièn 3,17-dione, 3 $cm^3$ d'orthochloroacétate de triéthyle (préparé comme indiqué dans JACS 64, 1825-7 (1942) et 10 μl d'acide propionique. On concentre partiellement sous pression réduite, élimine par filtration le précipité formé, chromatographie le filtrat sur silice (éluant : acétate d'éthyle-cyclohexane 3-7). On obtient 177 mg de produit attendu.

IR ($CHCl_3$) : 1739 $cm^{-1}$ (17-céto) ; 1672 $cm^{-1}$ (cétone conjuguée) ; 1636 et 1616 $cm^{-1}$ (C=C).

RMN ($CDCl_3$, 300 MHz) mélange 5-1 de 2 diastéréisomères: 0,93 et 0,94 (18 Me) ; 1,24 et 1,28 (2t, COOEt) ; 4,50 et 4,30 (COOEt) ; 4,89 et 4,99 (2s, CH(Cl)COOEt) ; 5,78 (m, H11) ; 5,92 et 6,01 (H4).

**Etude pharmacologique du produit de l'exemple 3 de l'invention.**

Inhibition dépendante de la concentration (mesure de la $CI_{50}$ = concentration de l'inhibiteur nécessaire pour réduire l'activité enzymatique de 50 %).

On utilise des placentas humains qui une heure au plus apres l'accouchement sont laves, perfuses par du serum physiologique (5 litres) via la veine ombilicale puis congelés à : -40 °C.

1) Obtention des microsomes placentaires

Les placentas sont décongelés à 4 °C puis homogénéisés (1:3) dans un tampon phosphate 10 mM, pH = 7,0; contenant 100 millimoles de chlorure de potassium (KC1), 10 millimoles de dithiothreitol (DTT), 10 millimoles d'acide éthylènediaminetétraacétique (EDTA), 40 millimoles de nicotinamide et 250 millimoles de sucrose.

Les homogénats sont ensuite soumis à différentes phases de centrifugation jusqu'à l'obtention du surnageant "9000 g" (correspondant au cytosol et au réticulum endoplasmique). Ce surnageant est alors soumis à une étape d'ultracentrifugation (1 heure 30 minutes, 105000 g) pour obtenir le culot microsomal.

Les microsomes sont alors resuspendus dans un tampon phosphate 50 millimoles, pH = 7,4, contenant 100 millimoles KC1, 1 millimole EDTA, 1 millimole DTT et du glycérol (10%).

La suspension microsomale est ensuite aliquotée et les fractions congelées à la température de l'azote liquide.

La concentration en protéines de la suspension microsomale est déterminée par la méthode de

BRADFORD (BRADFORD M., Anal. Biochem., 72, (1976) 248).

2) Mesure de la $CI_{50}$ de chaque inhibiteur

A 960 microlitres de tampon phosphate (50 millimoles, pH = 7,2), 2,5 millimoles glucose-6-phosphate, et contenant 0,16 unité internationale de glucose-6-phosphate déshydrogénase (G-6-PDH), on ajoute dans l'ordre:

1° - 10 microlitres d'inhibiteur, solubilisé dans le diméthylsulfoxyde (DMSO), pour donner des concentrations finales allant de $10^{-6}$ M à $10^{-9}$ M.

2° - 10 microlitres de substrat. Le substrat est de l'Androstènedione 60 nM solubilisée dans l'éthanol et contenant de la 1béta-2béta-($^3$H)-Androstènedione à dilution isotopique connue (≃ 200.000 désintégrations par minute).

3° - 10 microlitres de suspension microsomale, équivalant à 25 microgrammes de protéines par test.

La réaction enzymatique est alors très rapidement initiée par un ajout de 10 microlitres de nicotinamide adénine dinucléotide phosphate réduit (NADPH) solubilisé dans l'eau.

Après agitation, chaque test est incubé à 37°C pendant 10 minutes. La réaction est ensuite stoppée par un ajout de chloroforme (4 ml).

Après agitation vigoureuse des tubes, ceux-ci sont décantés et centrifugés à 4°C pendant 10 minutes à la vitesse de 3000 r.p.m. (rotations par minutes) soit 600 X g.

Après centrifugation, et pour chaque tube, 100 microlitres de surnageant sont prélevés et mis à compter en présence de liquide scintillant.

Cette méthode est dérivée des procédures décrites par REED et Coll. (J.Biol. Chem., 251, (1976), 1625) et THOMPSON et Coll. (J.Biol. Chem., 249, (1974), 5364).

L'activité enzymatique (aromatase), est proportionnelle au pourcentage de tritium relargué sous forme d'eau tritiée ($^3H_2O$) au cours de la réaction.

L'inhibition obtenue pour chaque concentration de chaque produit inhibiteur de l'invention est calculée comme un pourcentage des contrôles (100% arbitraire, obtenus en absence de tout inhibiteur).

La $CI_{50}$ est égale à la concentration d'inhibiteur nécessaire pour diminuer de 50% l'activité enzymatique.

Les valeurs des $CI_{50}$ obtenues pour les produits inhibiteurs de l'invention sont les suivantes:

Produit de l'exemple 3 : $CI_{50}$ = $8,5.10^{-8}$ M.

## Revendications

**1.** Les produits de formule générale (I) :

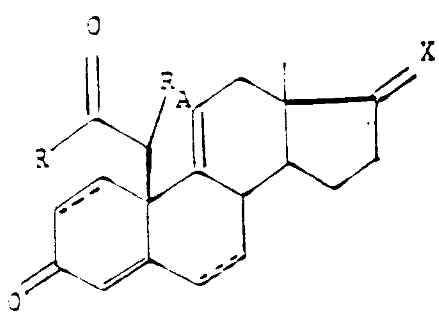

(I)

dans laquelle le substituant R représente :

- un atome d'hydrogène,
- un radical :

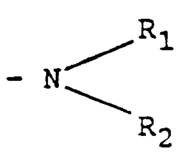

dans lequel $R_1$ et $R_2$ identiques ou différents représentent soit un radical alkyle ayant de 1 à 6 atomes de carbone soit $R_1$ et $R_2$ forment avec l'atome d'azote auquel ils sont liés un radical hétérocyclique à 5 ou 6 chaînons comportant éventuellement un second hétéroatome choisi

parmi les atomes d'oxygène, de soufre et d'azote,
- un radical alkyle ou alkyloxy ayant chacun de 1 à 12 atomes de carbone,
- un radical aryle, aralkyle, aryloxy ou (arylalkyl) oxy ayant chacun au plus 12 atomes de carbone,

$R_A$ représente un atome d'hydrogène, un atome d'halogène, un radical hydroxy, un radical alkyle, alkyloxy, alkylthio, amino, monoalkylamino, dialkylamino, dans chacun desquels alkyle renferment au plus 6 atomes de carbone, un radical carbamoyle, un radical alkoxycarbonyle renfermant au plus 7 atomes de carbone,

X représente un atome d'oxygène ou un radical

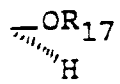

dans lequel $R_{17}$ représente un atome d'hydrogène ou un radical alkyle ou acyle ayant chacun au plus 12 atomes de carbone, et les traits pointillés en positions 1(2) et 6(7) indiquent la présence éventuelle d'une seconde liaison entre les carbones qui les portent, étant entendu que R représente un radical alkyloxy, aryloxy, ou (arylalkyl)oxy ayant au plus chacun 12 atomes de carbone lorsque $R_A$ ne représente pas un atome d'hydrogène.

**2.** Les produits de formule générale (I) telle que définie à la revendication 1, répondant à la formule (I') :

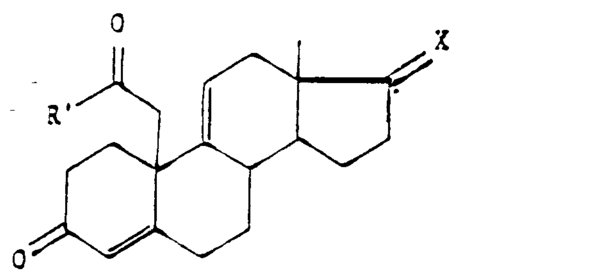

(I')

dans laquelle R' représente un atome d'hydrogène, un radical $-N(R'_1)_2$ dans lequel $R'_1$ représente un radical alkyle ayant de 1 à 4 atomes de carbone ou R' représente un radical alkyle ou alkyloxy ayant chacun de 1 à 4 atomes de carbone, X représente un atome d'oxygène ou un radical $^{OH}_{H}$

**3.** Le produit de formule (I'), telle que définie à la revendication 2 dont le nom suit :
- 3-17-dioxo androsta-4,9(11)-diène-19-carboxaldéhyde.

**4.** Procédé de préparation des produits de formule ($I_a$) correspondant aux produits de formule (I) telle que définie à la revendication 1, caractérisé en ce que l'on soumet un produit de formule (II) :

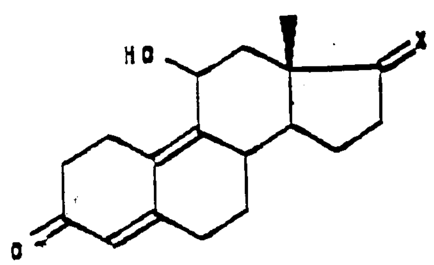

(II)

dans laquelle X a la signification indiquée ci-dessus à l'action d'un produit de formule (III) :

$R_A$-$CH_2$-$C(OR_a)_3$ (III)

dans laquelle $R_A$ a la signification indiquée à la revendication 1, $R_a$ représente un radical alkyle, aryle ou arylalkyle ayant chacun au plus 12 atomes de carbone, pour obtenir les produits de formule (Ia)

correspondant aux produits de formule (I) dans laquelle R représente un radical alkyloxy, aryloxy ou (arylalkyl) oxy ayant chacun au plus 12 atomes de carbone, $R_A$ a la signification indiquée à la revendication 1 et les traits pointillés en positions 1(2) et 6(7) ne représentent pas une seconde liaison entre les carbones qui les portent.

**5.** Procédé de préparation des produits de formule ($I_b$) correspondant aux produits de formule (I) telle que définie à la revendication 1, caractérisé en ce que l'on soumet un produit de formule (II) à l'action d'un produit de formule (IV) :

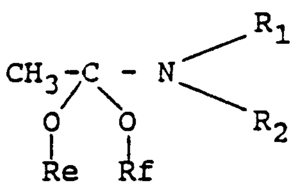

(IV)

dans laquelle $R_1$ et $R_2$ ont la signification indiquée à la revendication 1, Re et Rf identiques ou différents représentent un radical alkyle ayant chacun de 1 à 4 atomes de carbone, pour obtenir les produits de formule ($I_b$) correspondant aux produits de formule (I) dans laquelle R représente :

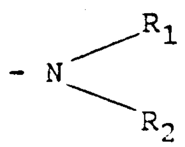

dans lequel $R_1$ et $R_2$ ont la signification indiquée ci-dessus, $R_A$ représente un atome d'hydrogène et les traits pointillés en position 1(2) et 6(7) ne représentent pas une seconde liaison entre les carbones qui les portent.

**6.** Procédé de préparation des produits de formule ($I_c$) correspondant aux produits de formule (I) telle que définie à la revendication 1, caractérisé en ce que l'on soumet un produit de formule (II) à l'action d'un produit de formule (V) :

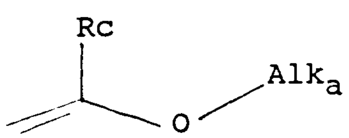

(V)

dans laquelle Rc représente un atome d'hydrogène ou un radical alkyle, aryle ou aralkyle ayant chacun au plus 12 atomes de carbone et $Alk_a$ représente un radical alkyle ayant de 1 à 4 atomes de carbone, pour obtenir les produits de formule (Ic) correspondant aux produits de formule (I) dans laquelle R représente un atome d'hydrogène ou un radical alkyle, aryle ou aralkyle ayant chacun au plus 12 atomes de carbone, $R_A$ représente un atome d'hydrogène et les traits pointillés en positions 1(2) et 6(7) ne représentent pas une seconde liaison entre les carbones qui les portent.

**7.** Procédé par lequel les produits de formule ($I_a$), dont la préparation est décrite dans la revendication 4, sont soumis à l'une ou plusieurs des réactions suivantes et dans un ordre quelconque :

- introduction d'une double liaison en position 1(2),
- introduction d'une double liaison en position 6(7),
- réduction de la fonction cétone en position 17 lorsque X représente un atome d'oxygène et formation d'un groupement 17$\beta$-OH,

- éthérification ou estérification des produits dans lesquels X représente un radical

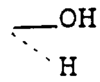

de façon à obtenir 17β-$OR_{17}$ dans lequel $R_{17}$ est un alkyle ou un acyle ayant chacun au plus 12 atomes de carbone.

**8.** Procédé par lequel les produits de formule ($I_b$), dont la préparation est décrite dans la revendication 5, sont soumis à l'une ou plusieurs des réactions suivantes et dans un ordre quelconque :
- introduction d'une double liaison en position 1(2),
- introduction d'une double liaison en position 6(7),
- réduction de la fonction cétone en position 17 lorsque X représente un atome d'oxygène, et formation d'un groupement 17β-OH,
- éthérification ou estérification des produits dans lesquels X représente un radical

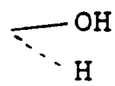

de façon à obtenir 17β-$OR_{17}$ dans lequel $R_{17}$ est un alkyle ou un acyle ayant chacun au plus 12 atomes de carbone.

**9.** Procédé par lequel les produits de formule ($I_c$), dont la préparation est décrite dans la revendication 6, sont soumis à l'une ou plusieurs des réactions suivantes et dans un ordre quelconque :
- introduction d'une double liaison en position 1(2)
- introduction d'une double liaison en position 6(7)
- réduction de la fonction cétone en position 17 lorsque X représente un atome d'oxygène, et formation d'un groupement 17β-OH,
- éthérification ou estérification des produits dans lesquels X représente un radical

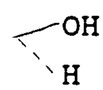

de façon à obtenir 17β-$OR_{17}$ dans lequel $R_{17}$ est un alkyle ou un acyle ayant chacun au plus 12 atomes de carbone.

**10.** A titre de médicament le produit tel que décrit à la revendication 3.

**11.** Les compositions pharmaceutiques contenant, à titre de principe actif le médicament défini à la revendication 10.

## Claims

**1.** The products of general formula (I):

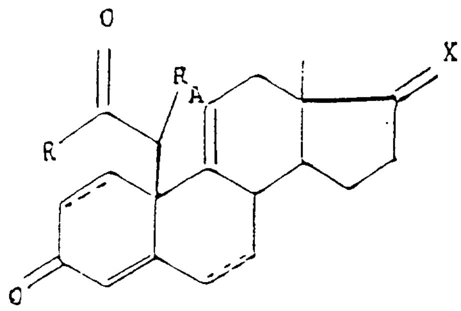

(I)

in which the R substituent represents:

- a hydrogen atom,
- a radical:

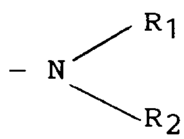

in which $R_1$ and $R_2$, identical or different, represent either an alkyl radical having 1 to 6 carbon atoms or $R_1$ and $R_2$ form with the nitrogen atom to which they are linked a heterocyclic radical with 5 or 6 members optionally containing a second heteroatom chosen from the oxygen, sulphur and nitrogen atoms,
- an alkyl or alkyloxy radical each having 1 to 12 carbon atoms,
- an aryl, aralkyl, aryloxy or (arylalkyl) oxy radical each having at most 12 carbon atoms,

$R_A$ represents a hydrogen atom, a halogen atom, a hydroxy radical, an alkyl, alkyloxy, alkylthio, amino, monoalkylamino, dialkylamino radical, in each of which the alkyl contains at most 6 carbon atoms, a carbamoyl radical, an alkoxycarbonyl radical containing at most 7 carbon atoms,

X represents an oxygen atom or an

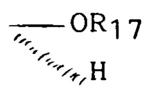

radical in which $R_{17}$ represents a hydrogen atom or an alkyl or acyl radical each having at most 12 carbon atoms, and the dotted lines in positions 1(2) and 6(7) indicate the optional presence of a second bond between the carbons which carry them, it being understood that R represents an alkyloxy, aryloxy, or (arylalkyl) oxy radical each having at most 12 carbon atoms when $R_A$ does not represent a hydrogen atom.

**2.** The products of general formula (I) as defined in claim 1, corresponding to the formula (I'):

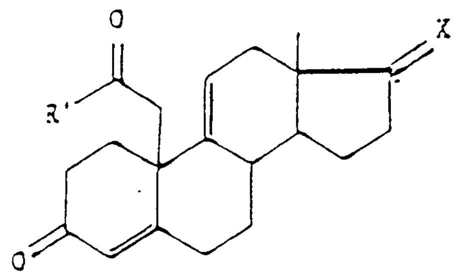

(I')

in which R' represents a hydrogen atom, an $-N(R'_1)_2$ radical in which $R'_1$ represents an alkyl radical having 1 to 4 carbon atoms or R' represents an alkyl or alkyloxy radical each having 1 to 4 carbon atoms,
X represents an oxygen atom or an

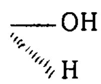

radical

**3.** The product of formula (I'), as defined in claim 2, the name of which follows:
- 3-17-dioxo androsta-4,9(11)-diene-19-carboxaldehyde.

**4.** Preparation process for the products of formula ($I_a$) corresponding to the products of formula (I) as defined in claim 1, characterized in that a product of formula (II):

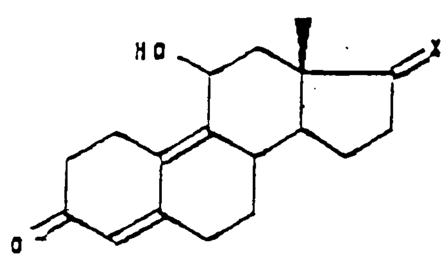

(II)

in which X has the meaning indicated above, is subjected to the action of a product of formula (III):

$R_A-CH_2-C(OR_a)_3$ (III)

in which $R_A$ has the meaning indicated in claim 1, $R_a$ represents an alkyl, aryl or arylalkyl radical each having at most 12 carbon atoms, in order to obtain the products of formula ($I_a$) corresponding to the products of formula (I) in which R represents an alkyloxy, aryloxy or (arylalkyl) oxy radical each having at most 12 carbon atoms, $R_A$ has the meaning indicated in claim 1 and the dotted lines in positions 1(2) and 6(7) do not represent a second bond between the carbons which carry them.

**5.** Preparation process for the products of formula ($I_b$) corresponding to the products of formula (I) as defined in claim 1, characterized in that a product of formula (II) is subjected to the action of a product of formula (IV):

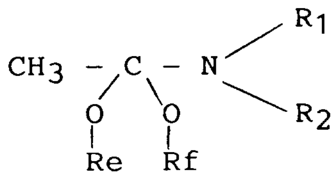

(IV)

in which $R_1$ and $R_2$ have the meaning indicated in claim 1, Re and Rf, identical or different, represent an alkyl radical each having 1 to 4 carbon atoms, in order to obtain the products of formula ($I_b$) corresponding to the products of formula (I) in which R represents:

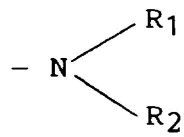

in which $R_1$ and $R_2$ have the meaning indicated above, $R_A$ represents a hydrogen atom and the dotted lines in position 1(2) and 6(7) do not represent a second bond between the carbons which carry them.

**6.** Preparation process for the products of formula ($I_c$) corresponding to the products of formula (I) as defined in claim 1, characterized in that a product of formula (II) is subjected to the action of a product of formula (V):

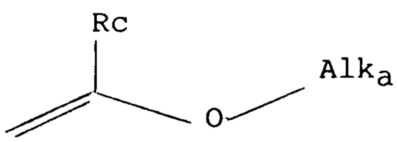

(V)

in which Rc represents a hydrogen atom or an alkyl, aryl or aralkyl radical each having at most 12 carbon atoms and $Alk_a$ represents an alkyl radical having 1 to 4 carbon atoms, in order to obtain the products of formula ($I_c$) corresponding to the products of formula (I) in which R represents a hydrogen atom or an alkyl, aryl or aralkyl radical each having at most 12 carbon atoms, $R_A$ represents a hydrogen atom and the dotted lines in positions 1(2) and 6(7) do not represent a second bond between the carbons which carry them.

**7.** Process by which the products of formula ($I_a$), whose preparation is described in claim 4, are subjected to one or more of the following reactions and in any order:

- introduction of a double bond in position 1(2),
- introduction of a double bond in position 6(7),
- reduction of the ketone function in position 17 when X represents an oxygen atom and formation of a 17β-OH group,
- etherification or esterification of the products in which X represents an

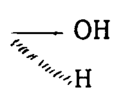

radical so as to obtain 17β-$OR_{17}$ in which $R_{17}$ is an alkyl or an acyl each having at most 12 carbon atoms.

**8.** Process by which the products of formula ($I_b$), whose preparation is described in claim 5, are subjected to one or more of the following reactions and in any order:

- introduction of a double bond in position 1(2),
- introduction of a double bond in position 6(7),

- reduction of the ketone function in position 17 when X represents an oxygen atom, and formation of a 17β-OH group,
- etherification or esterification of the products in which X represents an

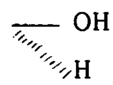

radical so as to obtain 17β-$OR_{17}$ in which $R_{17}$ is an alkyl or an acyl each having at most 12 carbon atoms.

**9.** Process by which the products of formula ($I_c$), whose preparation is described in claim 6, are subjected to one or more of the following reactions and in any order:
- introduction of a double bond in position 1(2)
- introduction of a double bond in position 6(7)
- reduction of the ketone function in position 17 when X represents an oxygen atom, and formation of a 17β-OH group,
- etherification or esterification of the products in which X represents an

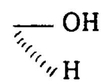

so as to obtain 17β-$OR_{17}$ in which $R_{17}$ is an alkyl or an acyl each having at most 12 carbon atoms.

**10.** As a medicament, the product as described in claim 3.

**11.** Pharmaceutical compositions containing, as active ingredient, the medicament defined in claim 10.

**Patentansprüche**

**1.** Die Produkte mit der allgemeinen Formel (I):

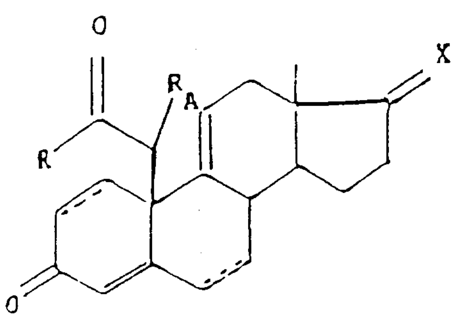

(I)

in der der Substituent R folgendes darstellt:
- ein Wasserstoffatom
- einen Rest:

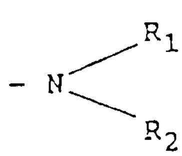

in dem $R_1$ und $R_2$, gleich oder verschieden, entweder für einen Alkylrest mit 1 bis 6 Kohlenstoff-

atomen stehen oder $R_1$ und $R_2$ mit den Stickstoffatom, an das sie gebunden sind, einen heterozyklischen Rest mit 5 oder 6 Kettengliedern bilden mit gegebenenfalls einem zweiten Heteroatom, ausgewählt aus dem Sauerstoff-, Schwefel- und Stickstoffatom,

- einen Alkyl- oder Alkoxyrest mit Jeweils 1 bis 12 Kohlenstoffatomen,
- einen Aryl- Aralkyl- Aryloxy- oder (Arylalkyl)oxyrest mit jeweils höchstens 12 Kohlenstoffatomen,

$R_A$ für folgendes steht: ein Wasserstoffatom, ein Halogenatom, einen Hydroxylrest, einen Alkyl-, Alkyloxy-, Alkylthio-, Amino-, Monoalkylamino- Dialkylaminorest, in denen die Alkyle jeweils höchstens 6 Kohlenstoffatome enthalten, einen Carbamoylrest, einen Alkoxycarbonylrest mit höchstens 7 Kohlenstoffatomen,

X für ein Sauerstoffatom oder einen Rest

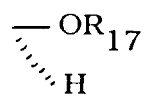

steht, in dem $R_{17}$ für ein Wasserstoffatom oder einen Alkyl- oder Acylrest mit jeweils höchstens 12 Kohlenstoffatomen steht und die punktierten Striche in den Positionen 1(2) und 6(7) die etwaige Anwesenheit einer zweiten Bindung zwischen den Kohlenstoffen anzeigen, die durch sie verbunden sind, wobei R für einen Alkoxy-, Aryloxy- oder (Arylalkyl)oxyrest mit höchstens 12 Kohlenstoffatomen steht, wenn $R_A$ kein Wasserstoffatom darstellt.

2. Die Produkte mit der allgemeinen Formel (I) wie in Anspruch 1 definiert, die folgender Formel (I') entsprechen:

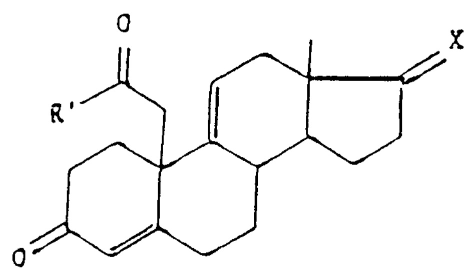

(I')

in der R' für ein Wasserstoffatom, einen -N(R'$_1$)$_2$-Rest steht, in dem R'$_1$ für einen Alkylest mit 1 bis 4 Kohlenstoffatomen steht oder R' für einen Alkyl- oder Alkoxyrest mit jeweils 1 bis 4 Kohlenstoffatomen steht,

X für ein Sauerstoffatom steht oder für einen Rest

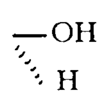

3. Das Produkt mit der Formel (I'), wie in Anspruch 2 definiert, mit folgendem Namen:
   - 3,17-Dioxoandrosta-4,9(11)-dien-19-carboxaldehyd.

4. Verfahren zur Herstellung der Produkte mit der Formel ($I_a$), die den Produkten mit der Formel (I) wie in Anspruch 1 definiert entsprechen, dadurch gekennzeichnet, daß man auf ein Produkt mit der Formel (II):

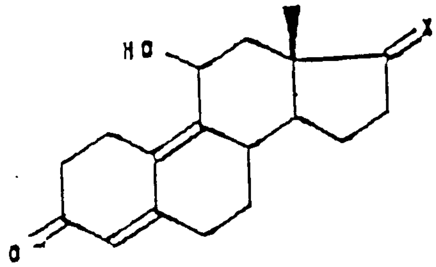

(II)

in dem X die oben angegebene Bedeutung hat, ein Produkt mit der Formel (III) einwirken läßt:

$R_A$-$CH_2$-$C(OR_a)_3$ (III)

in der $R_A$ die in dem Anspruch 1 angegebene Bedeutung hat, $R_a$ für einen Alkyl-, Aryl- oder Arylalkylrest mit jeweils mehr als 12 Kohlenstoffatomen steht, wodurch man Produkte mit der Formel (Ia) erhält, die den Produkten mit der Formel (I) entsprechen, in der R für einen Alkoxy-, Aryloxy- oder (Arylalkyl)oxyrest mit höchstens 12 Kohlenstoffatomen steht, $R_A$ die in Anspruch 1 angegebene Bedeutung hat und die punktierten Linien in den Positionen 1(2) und 6(7) nicht eine zweite Bindung zwischen den Kohlenstoffen, durch die sie verbunden sind, darstellen.

**5.** Verfahren zur Herstellung der Produkte mit der Formel ($I_b$), die den Produkten mit der Formel (I) entsprechen, wie sie in Anspruch 1 definiert ist, dadurch gekennzeichnet, daß man auf ein Produkt mit der Formel (II) ein Produkt mit folgender Formel (IV) einwirken läßt,

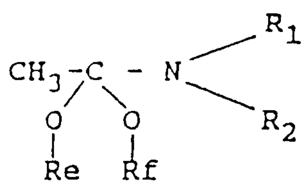

(IV)

in der $R_1$ und $R_2$ die in dem Anspruch 1 angegebene Bedeutung haben, Re und Rf, gleich oder verschieden, für einen Alkylrest mit jeweils 1 bis 4 Kohlenstoffatomen stehen, wodurch man Produkte mit der Formel ($I_b$) erhält, die den Produkten mit der Formel (I) entsprechen, in der R folgendes darstellt:

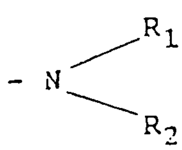

worin $R_1$ und $R_2$ die oben angegebene Bedeutung haben, $R_A$ für ein Wasserstoffatom steht und die punktierten Linien in Position 1(2) und 6(7) nicht eine zweite Bindung zwischen den Kohlenstoffatomen darstellen, die durch sie verbunden sind.

**6.** Verfahren zur Herstellung der Produkte mit der Formel ($I_c$), die den Produkten mit der Formel (I) wie in Anspruch 1 definiert entsprechen, dadurch gekennzeichnet, daß man auf ein Produkt mit der Formel (II) ein Produkt mit folgender Formel (V) einwirken läßt:

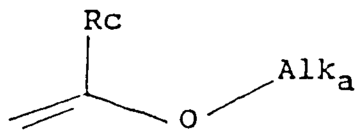

(V)

in der Rc für ein Wasserstoffatom steht oder für einen Alkyl-, Aryl- oder Aralkylrest mit jeweils höchstens 12 Kohlenstoffatomen und $Alk_a$ für einen Alkylrest mit 1 bis 4 Kohlenstoffatomen steht, wodurch man Produkte mit der Formel (Ic) erhält, die den Produkten mit der Formel (I) entsprechen, in der R für ein Wasserstoffatom steht oder für einen Alkyl-, Aryl- oder Aralkylrest mit jeweils höchstens 12 Kohlenstoffatomen, $R_A$ für ein Wasserstoffatom steht und die punktierten Linien in den Positionen 1-(2) und 6(7) nicht für eine zweite Bindung zwischen den Kohlenstoffen steht, die durch sie verbunden sind.

**7.** Verfahren, durch das die Produkte mit der Formel ($I_a$), deren Herstellung in Anspruch 4 beschrieben ist, einer oder mehrerer der folgenden Reaktionen in beliebiger Reihenfolge unterzogen werden:
- Einführung einer Doppelbindung in Position 1(2),
- Einführung einer Doppelbindung in Position 6(7),
- Reduktion der Ketonfunktion in Position 17, wenn X für ein Sauerstoffatom steht, und Bildung einer 17β-OH-Gruppierung,
- Veretherung oder Veresterung der Produkte, in denen X für einen Rest

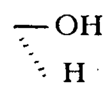

steht, um 17β-$OR_{17}$ zu erhalten, worin $R_{17}$ ein Alkyl oder Acyl mit jeweils höchstens 12 Kohlenstoffatomen ist.

**8.** Verfahren, durch das die Produkte mit der Formel ($I_b$), deren Herstellung in Anspruch 5 beschrieben ist, einer oder mehreren der folgenden Reaktionen in beliebiger Reihenfolge unterzogen werden:
- Einführung einer Doppelbindung in Position 1(2),
- Einführung einer Doppelbindung in Position 6(7),
- Reduktion der Ketonfunktion in Position 17, wenn X für ein Sauerstoffatom steht, und Bildung einer 17β-OH-Gruppierung,
- Veretherung oder Veresterung der Produkte, in denen X für einen Rest

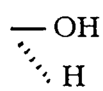

steht, um 17β-$OR_{17}$ zu erhalten, worin $R_{17}$ ein Alkyl oder Acyl mit jeweils höchstens 12 Kohlenstoffatomen ist.

**9.** Verfahren, durch das die Produkte mit der Formel ($I_c$), deren Herstellung in dem Anspruch 6 beschrieben ist, einer oder mehreren der folgenden Reaktionen in beliebiger Reihenfolge unterzogen werden:
- Einführung einer Doppelbindung in Position 1(2),
- Einführung einer Doppelbindung in Position 6(7),
- Reduktion der Ketonfunktion in Position 17, wenn X für ein Sauerstoffatom steht, und Bildung einer 17β-OH-Gruppierung,

- Veretherung oder Veresterung der Produkte, in denen X für einen Rest

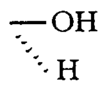

steht, um 17β-$OR_{17}$ zu erhalten, worin $R_{17}$ ein Alkyl oder Acyl mit jeweils höchstens 12 Kohlenstoffatomen ist.

**10.** Das Produkt, wie es in Anspruch 3 beschrieben ist, als Arzneimittel.

**11.** Die pharmazeutischen Zusammensetzungen, die als Wirkstoff das in Anspruch 10 definierte Arzneimittel enthalten.